# EUROPÄISCHE PATENTSCHRIFT

(11) **EP 0 897 311 B1**
(45) Veröffentlichungstag und Bekanntmachung des Hinweises auf die Patenterteilung: **13.09.2006**
(21) Anmeldenummer: 97954335.2
(22) Anmeldetag: 17.12.1997
(51) Int. Cl.: A61N 1/05, A61N 1/362

(54) **STIMULATIONSELEKTRODENANORDNUNG**
PACING LEAD SYSTEM
SYSTEME D'ELECTRODES DE STIMULATION

(30) Priorität: 17.12.1996 DE 19654491
(43) Veröffentlichungstag der Anmeldung: 24.02.1999
(60) Teilanmeldung: 03090211.8
(73) Patentinhaber: BIOTRONIK GmbH & Co. KG, 12359 Berlin (DE)
(72) Erfinder: THONG, Tran, Lake Oswego, OR 97035 (US); SCHALDACH, Max, D-91054 Erlangen (DE)
(74) Vertreter: Lindner-Vogt, Karin L.
(86) Internationale Anmeldenummer: PCT/DE1997/003024
(87) Internationale Veröffentlichungsnummer: WO 1998/026836

(56) Entgegenhaltungen:
- WO-A-97/31678
- DE-A- 19 507 929
- US-A- 4 394 866
- US-A- 5 235 977
- US-A- 5 314 430
- US-A- 5 376 103

## Beschreibung

Die Erfindung betrifft eine Stimulationselektrodenanordnung für einen Herzschrittmacher.

Bei der Therapie verschiedener chronischer Herzrhythmusstörungen sind seit langem implantierte Herzschrittmacher in Verbindung mit auf einem intrakardialen Elektrodenkatheter angeordneten und an der Herzinnenwand positionierten Stimulationselektroden in Gebrauch, über die das reizbare Herzgewebe erregt und dadurch ein Defekt des körpereigenen kardialen Reizbildungs- und -leitungssystems ausgeglichen wird.

Die Ausgestaltung der Schrittmacher und der zugehörigen Elektrodenleitungen hat zunehmende Vervollkommnung erfahren. Mit dem Ziel einer langfristigen Gewährleistung eines guten Kontaktes zwischen der oder den Stimulationselektrode(n) und dem Herzgewebe im Interesse einer zugleich energiesparenden und sicheren Stimulation sind zahlreiche technische Lösungen zur Verankerung der Elektrodenleitungen an der Herzwand - sowohl in der Herzkammer (Ventrikel) als auch im Vorhof (Atrium) - gefunden worden und tatsächlich wesentliche praktische Verbesserungen gelungen.

Dennoch zählen auch heute noch Elektrodendislokationen, die zu einer Verschlechterung oder einem Verlust des Kontakts mit dem reizbaren Gewebe an der Herzinnenwand und in der Folge zumindest zu einer wesentlichen Erhöhung der Reizschwelle und damit einem erhöhten Stromverbrauch des Schrittmachers und einer verringerten Lebensdauer und schlimmstenfalls zu dessen Funktionsunfähigkeit führen, zu den wichtigsten Komplikationen bei der Schrittmachertherapie. Die technische Weiterentwicklung von (immer komplizierter und aufwendiger werdenden) Verankerungsmechanismen dauert daher noch immer an.

Neben dem hohen Herstellungsaufwand ist bei diesen herkömmlichen Elektrodenanordnungen auch die recht aufwendige und große Erfahrung erfordernde Implantation von Nachteil.

Von vornherein nicht fest in der Herzkammer oder dem Vorhof zu verankernde (sogenannte "flottierende") und daher einfachere, kostengünstigere und leichter zu implantierende Elektrodenanordnungen sind - vorrangig aus implantierbaren Defibrillatoranordnungen, aber auch aus dem Einsatz mit Herzschrittmachern - ebenfalls bekannt.

Eine frühe Anordnung dieser Art zeigt die US-Patentschrift 3 915 174.

In der europäischen Patentanmeldung EP 0 601 338 A1 ist ein Elektrodensystem für einen implantierten Defibrillator beschrieben, das mindestens zwei - ohne besondere Verankerung allein aufgrund ihrer Größe ortsfest gehaltene - intravaskular plazierte Spulenelektroden (spiralförmige Elektroden) umfaßt. Eine dieser großflächigen Defibrillationselektroden kann speziell in der Vena cava superior angeordnet sein.

In der europäischen Patentanmeldung EP-0 677 301-Al ist ein Elektrodenkatheter zum Einsatz mit einem Defibrillator beschrieben, bei dem eine langgestreckte zylindrische Elektrode mit einer längs der Leitung verschiebbaren Abdeckung eine Verschiebung des wirksamen Elektrodenbereiches ermöglicht. Der Katheter kann beispielsweise so eingestellt sein, daß eine erste Elektrode im Ventrikel und der durch Verschiebung der Abdeckung positionierte Elektrodenbereich in der Vena cava superior positioniert ist.

Im Herzen flottierende Stimulationselektroden konnten bei klinischen Untersuchungen in der Vergangenheit nicht mit so hinreichender Sicherheit eine zuverlässige Stimulation mit den verfügbaren Reizimpulsspannungen und unter sparsamer Nutzung der begrenzten Batteriekapazität gewährleisten, daß eine nennenswerte praktische Anwendung hätte erfolgen können.

Das herkömmliche Vorgehen, die Stimulationsimpulse eines implantierten Schrittmachers über wandständige Elektroden an das reizbare Muskelgewebe der Herzinnenwandung im unteren Bereich des Ventrikels bzw. des Atriums zu übertragen, kann - ganz abgesehen von den angesprochenen praktischen Problemen - bei einer Reihe häufiger Herzrhythmusstörungen aus grundsätzlichen physiologischen Erwägungen nicht befriedigen: Mit diesem Vorgehen werden nämlich Bereiche auf den unteren hierarchischen Ebenen des kardialen Reizbildungs- und -leitungsystems stimuliert. Dies kann beispielsweise eine technisch aufwendige Zweikammerstimulation (im Atrium und Ventrikel) auch bei Krankheitsbildern erforderlich machen, für die ein Sinusknotendefekt prägend, bei denen das übrige Reizleitungssystem jedoch weitgehend intakt ist.

Die DE-Patentanmeldung 196 09 471.2 der Anmelderin betrifft eine Anordnung mit vorzugsweise flottierenden Stimulationselektroden unterschiedlicher Polarität zur Erzeugung eines auf vorbestimmte Herzgewebsbereiche "fokussierten" Reizimpulsfeldes.

In der US-A-5,235,977 ist eine Elektrodenanordnung für Defibrillierungszwecke angegeben, welche zwei intravaskuläre Elektroden und eine Flächenelektrode außerhalb des Herzens aufweist. Die intravaskulären Elektroden sind in der Vena Cava Superior angeordnet.

Die US-A-5,314,430 offenbart einen atriellen Defibrillationspulsgenerator mit einem Elektrodenableitsystem. Das Elektrodenableitsystem umfasst eine Koronarsinus-Elektrode und eine Subkutan-Elektrode, welche vorzugsweise auf dem Gehäuse des implantierbaren Defibrillators angebracht ist. Das Elektrodensystem kann auch zusätzliche Elektroden zur Anbringung im rechten Ventrikel und/oder in der Vena Cava Superior umfassen.

Der Erfindung liegt die Aufgabe zugrunde, eine verbesserte Elektrodenanordnung der eingangs genannten Gattung anzugeben, die einen hinsichtlich der Reizimpulslokalisierung besser den physiologischen Gegebenheiten folgenden und zuverlässigeren Betrieb eines Herzschrittmachers ermöglicht sowie einfach herzustellen und leicht und sicher zu positionieren ist.

Diese Aufgabe wird durch eine Stimulationselektrodenanordnung mit den Merkmalen des Anspruchs 1 gelöst.

Die Erfindung schließt den Gedanken ein, eine Elektrodenanordnung zu realisieren, mit der die Stimulationsimpulse des Schrittmachers den in der Reizbildungshierarchie hochstehenden Bereichen des kardialen Reizbildungs- und -leitungssystems - primär dem Sinusknoten - zugeführt werden können, um etwa einen Sinusknotendefekt auf möglichst einfache Weise mittels präzise lokalisierter elektrischer Stimulation am Ort des Defekts ausgleichen zu können.

Zur Ausführung dieses Gedankens bedient sich die vorgeschlagene Elektrodenanordnung einer Lokalisierung des aus einer einzelnen Stimulationsspannung erzeugten Reizimpulsfeldes im Sinusknoten bzw. einem sonstigen zentralen Bereich des Reizbildungssytems. Dazu dienen in herznahen Blutgefäßen und wahlweise im Vorhof in unmittelbarer Nachbarschaft dieses Bereiches (bevorzugt verankerungsfrei) angeordnete Stimulationselektroden.

Zumindest die erste Stimulationselektrode im herznahen Blutgefäß ist bevorzugt auf einer zur Erstreckung mindestens bis in den Vorhof und zur verankerungsfreien, insbesondere schwimmenden, Verlegung ausgebildeten Elektrodenleitung angeordnet.

Die zweite Stimulationselektrode ist in einer bevorzugten Fortbildung dieser Ausführung in solchem Abstand von der ersten Stimulationselektrode auf der Elektrodenleitung angeordnet, daß sie im implantierten Zustand im Vorhof positioniert ist.

Die Elektrodenleitung kann weiter eine oder auch mehrere distal von der ersten bzw. zweiten Stimulationselektrode derart beabstandete Elektrode(n) aufweisen, daß diese im implantierten Zustand in der Herzkammer angeordnet ist bzw. sind. Diese Ventrikelelektrode(n) kann/können insbesondere als Abfühlelektrode(n), bei speziellen Herzrhythmusstörungen - etwa vollständigem AV-Block - aber auch als zusätzliche Stimulationselektrode(n) dienen.

Die erste Stimulationselektrode - oder die Elektrodenleitung, falls eine solche vorgesehen ist - kann insbesondere Positionierungsmittel aufweisen, mittels derer eine vorbestimmte Position der ersten Stimulationselektrode in der Vena cava superior und/oder der zweiten Stimulationselektrode im Vorhof einstellbar ist.

Speziell kann die erste Stimulationselektrode selbst derart geformt sein, daß sie im implantierten Zustand eine vorbestimmte Position in der Vena cava superior einnimmt bzw. ggfs. auch der Vorhofelektrode eine bestimmte Lage verleiht - sofern sie in der Ausführung mit gemeinsamer Elektrodenleitung mit dieser mechanisch verbunden ist. Dies ist etwa durch eine wendel- oder wendelabschnittsförmige Ausführung mit annähernd dem Gefäßinnendurchmesser entsprechendem Wendeldurchmesser möglich.

Die erwähnten Positionierungsmittel sind in zweckmäßiger Weise als Teil(e) der Elektrodenleitung ausgebildet und umfassen in einer vielfältig einstellbaren Ausführung Mittel zur Einstellung einer vorbestimmten Krümmung der Elektrodenleitung im implantierten Zustand.

Alternativ oder zusätzlich hierzu können die Positionierungsmittel elektrisch isolierende oder von den Stimulationselektroden isolierte Abstandhalter umfassen.

Des weiteren können sie von außerhalb des Körpers betätigbare Mittel zur Veränderung der Position der ersten und/oder zweiten Stimulationselektrode im implantierten Zustand zur nachträglichen Lagekorrektur der Elektroden bezüglich des zu stimulierenden Herzgewebsbereiches aufweisen.

Ihrer Stimulationsfunktion entsprechend, sind die erste und/oder die zweite Stimulationselektrode ring- oder ringsegmentförmig mit einem Länge/Durchmesser-Verhältnis von maximal 2, bevorzugt von kleiner als 1, ausgebildet. (Unter diese Charakterisierung soll auch eine Ausbildung der ersten oder zweiten Elektrode als annähernd zylinder- bzw. "topf"förmige Spitzenelektrode am distalen Ende einer Zuleitung fallen.)

Weiterhin weisen die erste und/oder zweite Stimulationselektrode bevorzugt eine geometrische Oberfläche von weniger als 10 mm² und insbesondere eine fraktale Oberflächen-Mikrostruktur zur Vergrößerung der elektrisch wirksamen Oberfläche um einen Faktor von mindestens 10² auf.

Bei einer hinsichtlich der erreichbaren Reizimpulsfeldverteilung besonders variablen Ausführung ist als weitere Stimulationselektrode eine außerhalb des Herzens angeordnete indifferente Flächenelektrode vorgesehen, die insbesondere als Teil des Gehäuses des Herzschrittmachers realisiert sein kann.

Die präzise Lokalisierung eines hinreichend großen Potentialgradienten durch die beanspruchte Elektrodenanordnung ermöglicht die Überschreitung der Reizschwelle in durch die Elektroden nicht direkt kontaktierten Gewebsbereichen und damit die Reizung der nicht an der Oberfläche der Herzwand und zudem in Gebieten des Herzens, in denen eine aktive Fixierung von Elektroden praktisch unmöglich ist, liegenden Zielbereiche für die Erregung, nämlich des Sinusknotens oder ggfs. des AV-Knotens oder anderer hierarchisch relativ hochstehender Bereiche des Reizbildungs- und -leitungssystems.

Da eine Fixierung der Elektrodenleitung an der Herzwand bei der vorgeschlagenen Stimulationselektrodenanordnung nicht erforderlich ist, kann diese im Herzen "schwimmen". Sie wird mithin zweckmäßigerweise mit einer solchen Biegesteifigkeit aufgebaut und gegebenenfalls. einer solchen vorgeprägten Krümmung im Längsverlauf versehen, daß die Elektroden nach Einführung nicht in direktem Kontakt mit der Gefäß- bzw. Vorhofwandung stehen, aber nahe dem zu reizenden Bereich angeordnet sind.

Die Krümmung bzw. das Abstehen von Abstandhaltern von der Elektrodenleitung werden durch geeignete (als solche bekannte) Mittel nach der Einführung erzeugt, bevorzugt etwa durch Elemente aus einer bei Körpertemperatur aktivierten Gedächtnislegierung oder Vorspannelemente, die bei Entfernung eines zum Einführen dienenden Führungsdrahtes aus der Elektrodenleitung eine vorgeprägte Form annehmen. Es ist auch möglich, Mittel zur Veränderung der Krümmung von außen vorzusehen, etwa einen ähnlich wie ein Führungsdraht wirkenden Versteifungsdraht.

Bei einer anderen vorteilhaften Ausführungsform sind unterschiedliche Elektrodenbereiche, welche entweder auf der Elektrodenleitung angeordnet sind oder durch das Schrittmachergehäuse gebildet werden gleichzeitig oder alternativ einschaltbar, so daß eine unterschiedliche Feldverteilung, insbesondere auch durch ferngesteuert-programmierte Umschaltung, erzielbar ist. Auf diese Weise kann unterschiedlichen physiologischen Anforderungen Rechnung getragen werden. Für das Atrium sind trotzdem lediglich zwei (interne bzw. externe Anschlußleitungen des Stimulators erforderlich.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine schematische Darstellung des Reizbildungs- und Erregungsleitungssystems des Herzens,
Figur 2 eine schematische Darstellung einer Stimulationselektrodenanordnung gemäß einer Ausführungsform der Erfindung,
Figur 3 eine schematische Darstellung einer anderen Stimulationselektrodenanordnung.

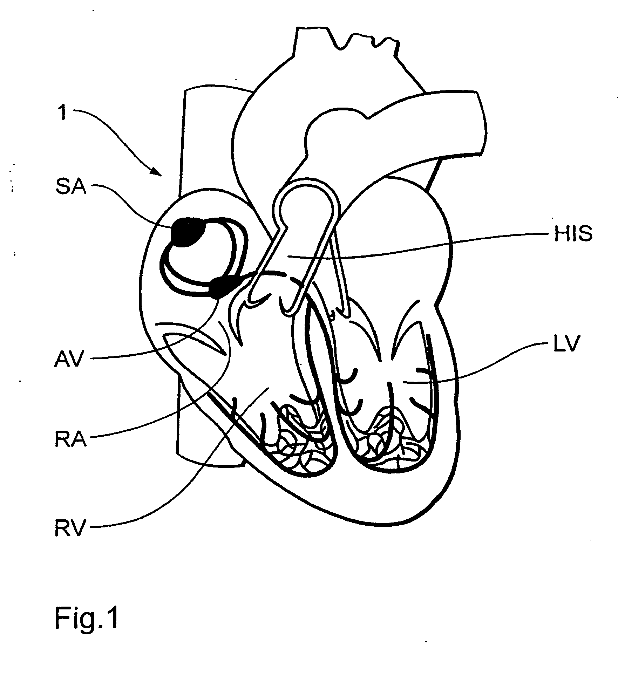

Die Lage der Elemente des Reizbildungs- und Erregungsleitungssystems im Herzen 1, wozu auch das oben nicht erwähnte His-Bündel und der jeweils in die Purkinje-Fasern mündende linke bzw. rechte Schenkel ("Left" bzw. "Right Bundle Branch") gehören, ist zunächst in Fig. 1 skizzenartig gezeigt. Es ist zu ersehen, daß mit der üblichen Stimulation über wandständig verankerte Elektroden in den unteren Regionen der Herzkammer (Ventrikel - LV bzw. RV) und/oder im Vorhof (Atrium - RA) lediglich periphere Bereiche des Systems erregt werden können, so daß auf Defekte in zentraleren Bereichen, etwa dem Sinusknoten (SA) oder AV-Knoten (AV), nicht direkt Einfluß genommen werden kann.

Nachfolgend werden ausgewählte Beispiele für konkrete Stimulationselektrodenanordnungen angegeben, die vom Grundgedanken der Erfindung Gebrauch machen, jedoch nicht im Sinne einer Beschränkung zu verstehen sind. Der Fachmann kann unter Rückgriff auf Details bekannter Elektrodenleitungen ohne weiteres eine Vielzahl weiterer Konfigurationen ausführen, die ebenfalls in den Schutzbereich der Ansprüche fallen.

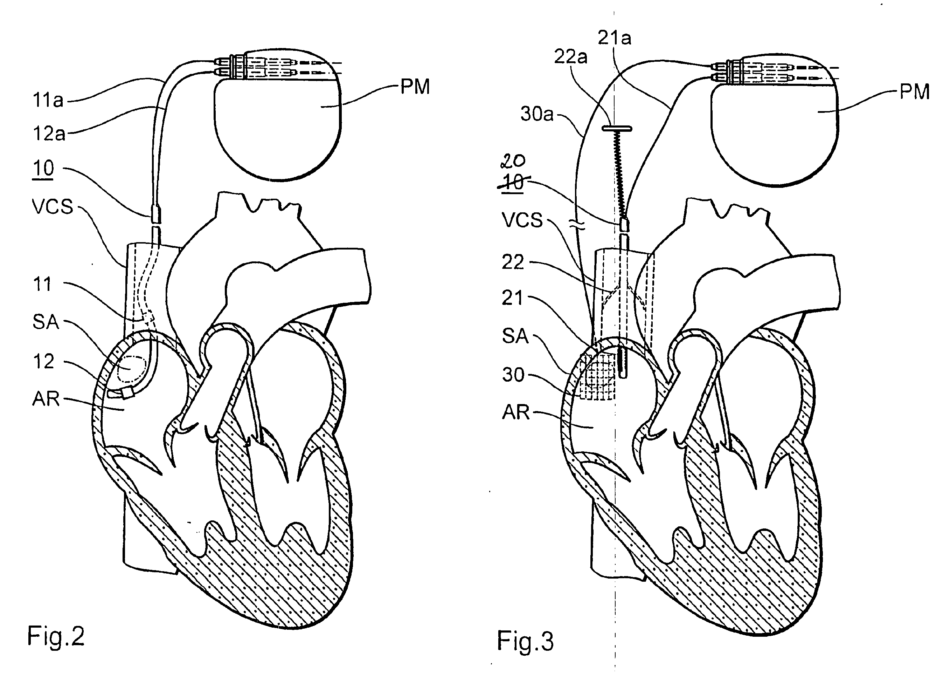

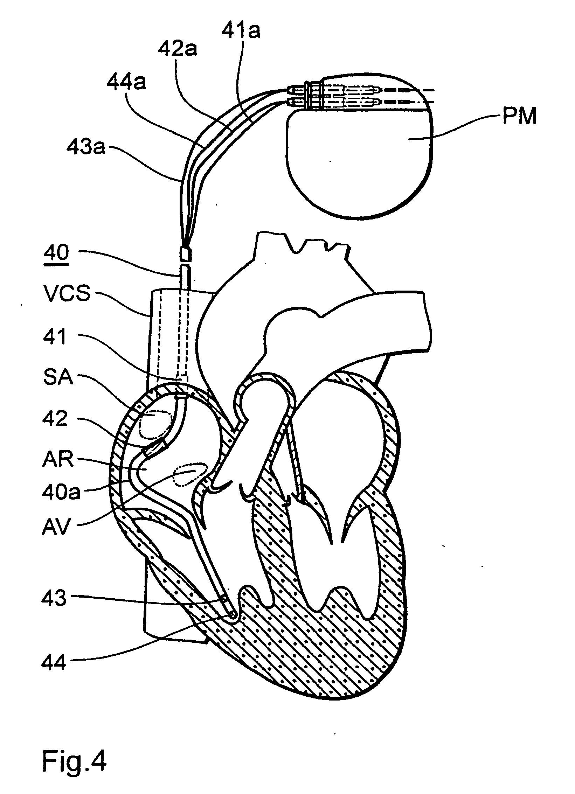

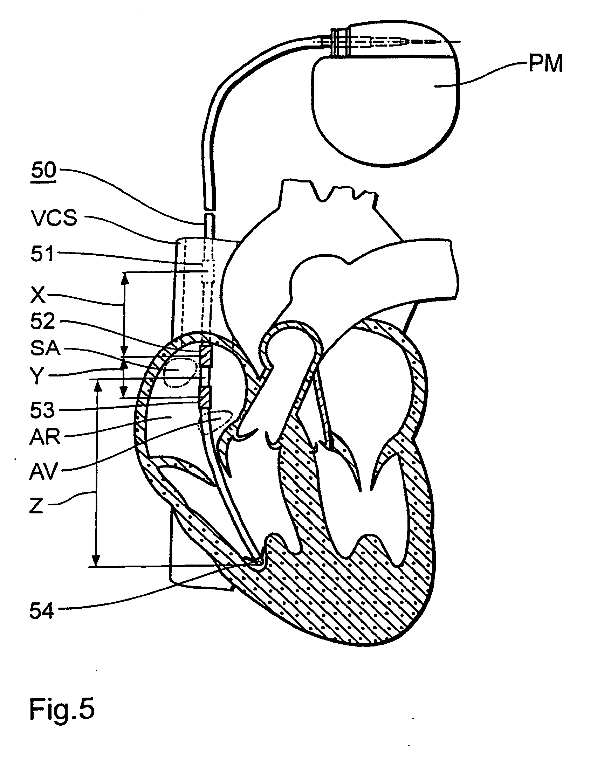

Fig. 2 ist eine schematische Darstellung einer mit S-förmig gekrümmtem Verlauf über die Vena cava superior VCS schwimmend in das rechte Atrium AR des Herzens H verlegte Elektrodenleitung 10. Auf der Leitung 10 sind eine erste, im dargestellten implantierten Zustand in der Vena cava positionierte Stimulationselektrode 11 und mit geringem Abstand zum distalen Leitungsende eine zweite, im hohen Atrium positionierte Stimulationselektrode 12 gebildet. Beide Elektroden 11, 12 sind als Ringelektroden mit (was in der Figur nicht erkennbar ist) fraktalartig mikrostrukturierter Oberfläche ausgeführt. Der mit vorgeprägter Krümmung ausgeführte Leitungskörper wird für die Einführung in bekannnter Weise durch einen (nicht gezeigten) Führungsdraht gestreckt und bei der Implantation in eine solche Winkelstellung gedreht, daß die Elektroden den Sinusknoten SA in möglichst geringem Abstand im wesentlichen zwischen sich einschließen. Die Elektroden 11, 12 sind über Zuleitungen 11a, 12a zu bipolarem Betrieb mit einem Herzschrittmacher PM verbunden und stimulieren bei Ausgabe eines Stimulationsimpulses an den Schrittmacherausgängen direkt den Sinusknoten SA.

Fig. 3 ist eine entsprechende Darstellung einer mir dem distalen Ende bis in den Übergangsbereich zwischen Vena cav superior VCS und rechtem Atrium AR reichenden Elektrodenleitung 20, welche an ihrem distalen Ende eine im Querschnitt halbkreisförmige Elektrode 21 aufweist. Diese ist über eine Zuleitung 21a mit einem Pol eines Herzschrittmachers PM verbunden, an dessen anderen Ausgang eine extrakardial, aber nahe dem Sinusknoten SA implantierte, relativ kleine Flächenelektrode 30 angeschlossen ist. (Je nach patientenspezifischer Lage des Sinusknotens ist ggfs. eine Einführung der zweiten Elektrode in ein herznahes Gefäß möglich und zu bevorzugen, da diese den Operationsaufwand verringert.) Die Position der Leitung 20 und der ersten Elektrode 21 im Gefäß SVC und damit relativ zum Sinusknoten SA wird durch von außen über einen Betätigungsgriff 22a gesteuert abspreizbare Abstandshalter 22 eingestellt. Auch bei dieser Anordnung bildet sich - korrekte Positionierung der Elektroden 21, 30 vorausgesetzt - bei Ausgabe eines Reizimpulses durch den Schrittmacher PM ein den Sinusknoten durchsetzendes Reizimpulsfeld aus.

Unter Fortlassung der extrakardialen Elektrode 30 kann mit der in Fig. 3 gezeigten Elektrodenleitung 20 grundsätzlich auch eine unipolare Stimulation gegen des Gehäuse des Schrittmachers PM als Referenz ausgeführt werden, was - um den Preis eines tendentiell höheren Energieverbrauchs und etwas erhöhter Störanfälligkeit - den technischen und operativen Aufwand verringert. Des weiteren können mit dem Schrittmachergehäuse als Referenzelektrode die bipolaren Anordnungen aus Fig. 2 und 3 zu tripolaren Stimulationsanordnungen ergänzt werden, was eine weiter verfeinerte Steuerung der Feldverteilung ermöglicht.

Es ist ersichtlich, daß der mit der Erfindung beabsichtigte Effekt auch mit Gruppen aus mehreren Elektroden erreicht werden kann. So können auch zwei oder mehr Elektroden im Atrium angeordnet oder aber auch eine weitere Elektrode in der Vena cava superior gelegen sein. Der Einsatz der einen oder der anderen Elektrodenanordnung kann von der tatsächlichen Lage des Sinusknotens bei einem Patienten und von dessen Reizschwelle abhängen.

Mit ganz ähnlichen Anordnungen wie den oben beschriebenen ist auch eine Reizimpulsfeldlokalisierung im AV-Knoten (vgl. Fig. 1) oder einem anderen zentralen Bereich des Reizbildungs- und -leitungssystems erreichbar.

## Patentansprüche

1. Stimulationselektrodenanordnung zur Stimulation des Herzens (H) mittels eines implantierbaren Herzschrittmachers (PM),
mit einer über die Vena Cava Superior (VCS) schwimmend in das rechte Atrium (AR) des Herzens verlegbaren Elektrodenleitung (10) mit einer auf der Elektrodenleitung (10) gebildeten, im implantierten Zustand in der Vena Cava Superior (VCS) positionierten ersten Stimulationselektrode (11) und einer ebenfalls auf der Elektrodenleitung (10) gebildeten, im implantierten Zustand im hohen Vorhof (AR) positionierten, derart angeordneten zweiten Stimulationselektrode (12), dass die erste und die zweite Stimulationselektrode den Sinusknoten (SA), im wesentlichen zwischen sich einschließen, wobei die erste Stimulationselektrode (21; 41) Positionierungsmittel (22; 40a) aufweist oder ihr solche zugeordnet sind, mittels derer eine vorbestimmte Position der ersten Stimulationselektrode in der Vena Cava Superior und/oder der zweiten Stimulationselektrode im Vorhof einstellbar ist,
**dadurch gekennzeichnet, dass**
die Positionierungsmittel (22; 40a) derart ausgebildet sind, dass die auf der schwimmend in das rechte Atrium(AR)des Herzens verlegbaren Elektrodenleitung (10) angeordneten Elektroden (11, 12; 21) nach Einführung der Elektrodenleitung (10) in das rechte Atrium (AR) des Herzens nicht im direkten Kontakt mit der Gefäß- bzw. Vorhofwandung stehen,
die jeweilige, auf der Elektrodenleitung am weitesten distal angeordnete Elektrode (12; 21) einen Abstand zum distalen Leitungsende hat, und die erste und/oder zweite Stimulationelektrode (11, 12 ; 21, 41, 42) ring - oder ringsegmentförmig mit einem Länge/Durchmesser - Verhältnis von maximal 2, bevorzugt von kleiner als 1, ausgebildet ist.

2. Stimulationselektrodenanordnung nach Anspruch 1, **dadurch gekennzeichnet, dass** mehrere auswählbar alternativ oder gemeinsam zuschaltbare zweite Stimulationselektroden (52; 53) vorgesehen sind.

3. Stimulationselektrodenanordnung nach Anspruch 1, **dadurch gekennzeichnet, dass** die Stimulationselektrodenanordnung mit einem Herzschrittmachergehäuse als Referenzelektrode eine tripolare Anordnung bildet.

4. Stimulationselektrodenanordnung nach Anspruch 1, **dadurch gekennzeichnet, dass** die erste Stimulationselektrode selbst derart geformt oder mit einem derart geformten Abschnitt der Leitung verbunden ist, dass sie im implantierten Zustand eine vorbestimmte Position in der Vena cava superior einnimmt.

5. Stimulationselektrodenanordnung nach Anspruch 3, **dadurch gekennzeichnet, dass** die Positionierungsmittel Mittel (40a) zur Einstellung einer vorbestimmten Krümmung der Elektrodenleitung (40) im implantierten Zustand umfassen.

6. Stimulationselektrodenanordnung nach Anspruch 1, **dadurch gekennzeichnet, dass** die Positionierungsmittel elektrisch isolierende oder von den Stimulationselektroden isolierte Abstandhalter (22) umfassen.

7. Stimulationselektrodenanordnung nach einem der Ansprüche 1, 2, 5 oder 6, **dadurch gekennzeichnet, dass** die Positionierungsmittel von außerhalb des Körpers betätigbare Mittel (22a) zur Veränderung der Position der ersten und/oder zweiten Stimulationselektrode im implantierten Zustand aufweisen.

8. Stimulationselektrodenanordnung nach Anspruch 1, **dadurch gekennzeichnet, dass** die erste und/oder zweite Stimulationselektrode (11, 12; 21; 41, 42) eine geometrische Oberfläche von weniger als 10 mm² und insbesondere eine fraktale Oberflächen-Mikrostruktur zur Vergrößerung der elektrisch wirksamen Oberfläche um einen Faktor von mindestens 10² aufweist.

## Claims

1. A stimulation electrode system for stimulating the heart (H) using an implantable cardiac pacemaker (PM),
having an electrode line (10), which may be laid floating in the right atrium (AR) of the heart via the vena cava superior (VCS), having a first stimulation electrode (11), which is formed on the electrode line (10) and is positioned in the vena cava superior (VCS) in the implanted state, and a second stimulation electrode (12), which is also formed on the electrode line (10) and is positioned in the high atrium (AR) in the implanted state, which is situated in such a way that the first and the second stimulation electrodes essentially enclose the sinus node (SA) between them, the first stimulation electrode (21; 41) having positioning means (22; 40a) or positioning means being assigned thereto, using which a predetermined position of the first stimulation electrode in the vena cava superior and/or the second stimulation electrode in the atrium may be set,
**characterized in that**
the positioning means (22; 40a) are implemented in such way that the electrodes (11, 12; 21), which are situated on the electrode line (10), which may be laid floating in the right atrium (AR) of the heart, are not in direct contact with the vascular or atrial wall after insertion of the electrode line (10) into the right atrium (AR),
the particular electrode (12; 21) situated furthest distal on the electrode line is at a distance to the distal line end, and the first and/or second stimulation electrode (11, 12; 21, 41, 42) is/are implemented in the shape of a ring or ring segment having a length/diameter ratio of at most 2, preferably less than 1.

2. The stimulation electrode according to Claim 1, **characterized in that** multiple second stimulation electrodes (52; 53), which may be selected alternately or interconnected jointly, are provided.

3. The stimulation electrode according to Claim 1, **characterized in that** the stimulation electrode system, using a cardiac pacemaker housing as the reference electrode, forms a tripolar system.

4. The stimulation electrode system according to Claim 1, **characterized in that** the first stimulation electrode itself is formed in such way or it is connected to a section of the line which is formed in such way that it occupies a predetermined position in the vena cava superior in the implanted state.

5. The stimulation electrode system according to Claim 3, **characterized in that** the positioning means comprise means (40a) for setting a predetermined curvature of the electrode line (40) in the implanted state.

6. The stimulation electrode system according to Claim 1, **characterized in that** the positioning means comprise spacers (22), which are electrically insulating or insulated from the stimulation electrodes.

7. The stimulation electrode system according to one of Claims 1, 2, 5, or 6, **characterized in that** the positioning means have means (22a), which may be actuated from outside the body, for changing the position of the first and/or second stimulation electrode in the implanted state.

8. The stimulation electrode system according to Claim 1, **characterized in that** the first and/or second stimulation electrode (11, 12; 21; 41, 42) has a geometric surface of less than 10 mm² and in particular a fractal surface microstructure to enlarge the electrically active surface by a factor of at least 10².

## Revendications

1. Dispositif d'électrodes de stimulation pour la stimulation du coeur (H) au moyen d'un pacemaker (PM) implantable,
comprenant une ligne d'électrodes (10) pouvant être posée dans l'oreillette droite (AR) du coeur de façon flottante au-dessus de la veine cave supérieure (VCS), une première électrode de stimulation (11) formée sur la ligne d'électrodes (10) et positionnée dans l'état implanté dans la veine cave supérieure (VCS) et une seconde électrode de stimulation (12) également formée sur la ligne d'électrodes (10) et positionnée dans l'état implanté dans l'oreillette (AR) supérieure, disposée de telle sorte que la première et la seconde électrodes de stimulation forment sensiblement entre elles le noeud sinusal (SA), la première électrode de stimulation (21 ; 41) présentant des moyens de positionnement (22 ; 40a) ou de tels moyens étant attribués à l'électrode, au moyen desquels une position prédéfinie de la première électrode de stimulation peut être réglée dans la veine cave supérieure et/ou une position prédéfinie de la seconde électrode de stimulation peut être réglée dans l'oreillette,
**caractérisé en ce que**
les moyens de positionnement (22 ; 40a) sont réalisés de telle sorte que les électrodes (11, 12 ; 21) disposées sur la ligne d'électrodes (10) pouvant être posée de façon flottante dans l'oreillette droite (AR) du coeur ne se trouvent pas, après l'introduction de la ligne d'électrodes (10) dans l'oreillette droite (AR) du coeur, en contact direct avec la paroi du vaisseau ou la paroi de l'oreillette,
l'électrode (12 ; 21) concernée, disposée la plus loin possible au plan distal sur la ligne d'électrodes, présente une distance à l'extrémité de ligne distale, et la première et/ou la seconde électrode de stimulation (11, 12 ; 21, 41, 42) est réalisée avec une forme d'anneau ou de segment d'anneau avec un rapport longueur/diamètre d'au maximum 2, de préférence inférieur à 1.

2. Dispositif d'électrodes de stimulation selon la revendication 1, **caractérisé en ce que** plusieurs secondes électrodes de stimulation (52 ; 53) pouvant être commutées au choix de façon alternative ou ensemble sont prévues.

3. Dispositif d'électrodes de stimulation selon la revendication 1, **caractérisé en ce que** le dispositif d'électrodes de stimulation forme avec un boîtier de pacemaker comme électrode de référence un agencement tripolaire.

4. Dispositif d'électrodes de stimulation selon la revendication 1, **caractérisé en ce que** la première électrode de stimulation est formée ou est reliée à une partie formée de la ligne de telle sorte qu'elle occupe dans l'état implanté une position prédéfinie dans la veine cave supérieure.

5. Dispositif d'électrodes de stimulation selon la revendication 3, **caractérisé en ce que** les moyens de positionnement comprennent des moyens (40a) pour le réglage d'une courbure prédéfinie de la ligne d'électrodes (40) dans l'état implanté.

6. Dispositif d'électrodes de stimulation selon la revendication 1, **caractérisé en ce que** les moyens de positionnement comprennent des écarteurs (22) électro-isolants ou isolés des électrodes de stimulation.

7. Dispositif d'électrodes de stimulation selon l'une quelconque des revendications 1, 2, 5 ou 6, **caractérisé en ce que** les moyens de positionnement présentent des moyens (22a) pouvant être actionnés à l'extérieur du corps pour la modification de la position de la première et/ou de la seconde électrode de stimulation dans l'état implanté.

8. Dispositif d'électrodes de stimulation selon la revendication 1, **caractérisé en ce que** la première et/ou la seconde électrode de stimulation (11, 12 ; 21 ; 41, 42) présente une surface géométrique de moins de 10 mm² et en particulier une microstructure de surface fractale pour l'agrandissement de la surface efficace électriquement d'un facteur d'au moins 10².
